# EUROPEAN PATENT APPLICATION

(11) **EP 2 952 947 A1**
(43) Date of publication of application: **09.12.2015**
(21) Application number: 14746497.8
(22) Date of filing: 28.01.2014
(51) Int. Cl.: G02B 23/26, A61B 1/00

(54) **OPTICAL SCANNING OBSERVATION DEVICE**

(30) Priority: 29.01.2013 JP 2013014819
(71) Applicant: Olympus Corporation, Shibuya-ku Tokyo 151-0072 (JP)
(72) Inventor: INNAMI, Takeharu, Tokyo 151-0072 (JP)
(74) Representative: Schicker, Silvia
(86) International application number: PCT/JP2014/000428
(87) International publication number: WO 2014/119288

(57) **Abstract**

An optical scanning observation apparatus (10) has an optical fiber (19), a vibratory drive unit (14), and a controller (15). The optical fiber (19) has an oscillating part supported at one end in an oscillatable manner. The vibratory drive unit (14) vibrates the oscillating part, based on a drive signal. The controller (15) changes the frequency of the drive signal to be transmitted to the vibratory drive means (14).

## Description

### CROSS REFERENCE TO RELATED APPLICATIONS

The present application claims the priority from Japanese Patent Application No. 2013-14819 filed on January 29, 2013, the entire disclosures of which is incorporated herein by reference.

### TECHNICAL FIELD

This disclosure relates to an optical scanning observation apparatus capable of suppressing variation in angle of view relative to ambient temperature variations.

### BACKGROUND

There has been known an optical scanning observation apparatus which vibrates an optical fiber emitting illumination light to thereby scan an observation object, so as to obtain an image of the observation object. In order to obtain an image suited for observation, it is necessary to vibrate the optical fiber along a predetermined vibration path.

Some of the optical scanning observation apparatuses, such as an remote visual inspection (RVI), may be used in a variety of external environments. In some environments, the optical fiber may deviate from a predetermined vibration path, which may generate distortion in an image to be obtained.

In view of the above, there has been proposed an endoscope apparatus for remapping an image having distortion under high temperatures, to thereby reduce distortion (see Patent Literature 1).

### CITATION LIST

### Patent Literature

PTL 1: JP 2011-004920 A

### SUMMARY

### (Technical Problem)

Changes in the external environment such as temperature variations not only generate distortion but may also cause variation in angle of view. The endoscope apparatus of PTL 1 is capable of correcting such distortion that may result from temperature variations, but could not suppress variation in angle of view.

In view of the aforementioned perspective, it could be helpful to provide an optical scanning observation apparatus capable of suppressing variation in angle of view, irrespective of changes in the external environment.

### (Solution to Problem)

In order to solve the aforementioned problems, an optical scanning observation apparatus according to a first aspect of this disclosure includes:
an optical fiber having an oscillating part supported at one end in an oscillatable manner;
vibratory drive means for vibrating the oscillating part, based on a drive signal; and
changing means for changing the frequency of the drive signal to be transmitted to the vibratory drive means.

Further, the optical scanning observation apparatus according to a second aspect of this disclosure, further includes a temperature sensor for detecting a temperature in the vicinity of the oscillating part,
in which the changing means may preferably change the frequency of the drive signal, based on the temperature detected by the temperature sensor

Further, the optical scanning observation apparatus according to a third aspect of this disclosure, further includes memory means for storing the correspondence of the frequency to the temperature in the vicinity of the oscillating part,
in which the changing means may preferably read out, from the memory means, a frequency corresponding to the temperature detected by the temperature sensor, and makes the frequency of the drive signal consistent with the frequency thus read out.

Further, in the optical scanning observation apparatus according to a fourth aspect of this disclosure, the optical fiber may preferably have: a core including at least one of pure silica, fluorine-doped silica, and hydroxyl-doped silica; a clad including at least one of pure silica, fluorine-doped silica, and hydroxyl-doped silica; a coating including at least one of a polyimide resin, a polyethyletherketone resin, and an acrylate resin; and a protection jacket including at least one of a polyimide resin, a polyethyletherketone resin, and an acrylate resin.

Further, the optical scanning observation apparatus according to the fifth aspect of this disclosure may preferably further include a detection optical fiber having: a core including at least one of pure silica, fluorine-doped silica, and hydroxyl-doped silica; a clad including at least one of pure silica, fluorine-doped silica, and hydroxyl-doped silica; a coating including at least one of a polyimide resin, a polyethyletherketone resin, and an acrylate resin; and a protection jacket including at least one of a polyimide resin, a polyethyletherketone resin, and an acrylate resin,
the detection optical fiber propagating light resulting from an interaction between light emitted from the oscillating part and the observation object.

### (Advantageous Effect)

According to the optical scanning observation apparatus disclosed herein, variations in the angle of view can be suppressed, irrespective of changes in the external environment.

### BRIEF DESCRIPTION OF THE DRAWINGS

In the accompanying drawings:
FIG. 1 is a functional block diagram schematically illustrating an internal configuration of an optical scanning observation apparatus according to Embodiment 1;
FIG. 2 is an overview schematically illustrating the optical scanning endoscope main body of FIG. 1;
FIG. 3 is an enlarged sectional view of a tip part of the optical scanning endoscope main body;
FIG. 4 is an enlarged perspective view of the scanning unit of FIG. 3;
FIG. 5 is a sectional view of an illumination optical fiber;
FIG. 6 is a graph illustrating the relation of the amplitude of the oscillating part to the frequency of the drive signal;
FIG. 7 is a graph showing the relation of the amplitude of the oscillating part to the frequency of the drive signal, for each temperature in the vicinity of the oscillating part;
FIG. 8 is a flowchart showing the processing to be carried out by the controller for determining the frequency in Embodiment 1; and
FIG. 9 is a functional block diagram schematically illustrating an internal configuration of an optical scanning observation apparatus according to Embodiment 2.

### DETAILED DESCRIPTION

In the following, Embodiments of this disclosure are disclosed with reference to the accompanying drawings.

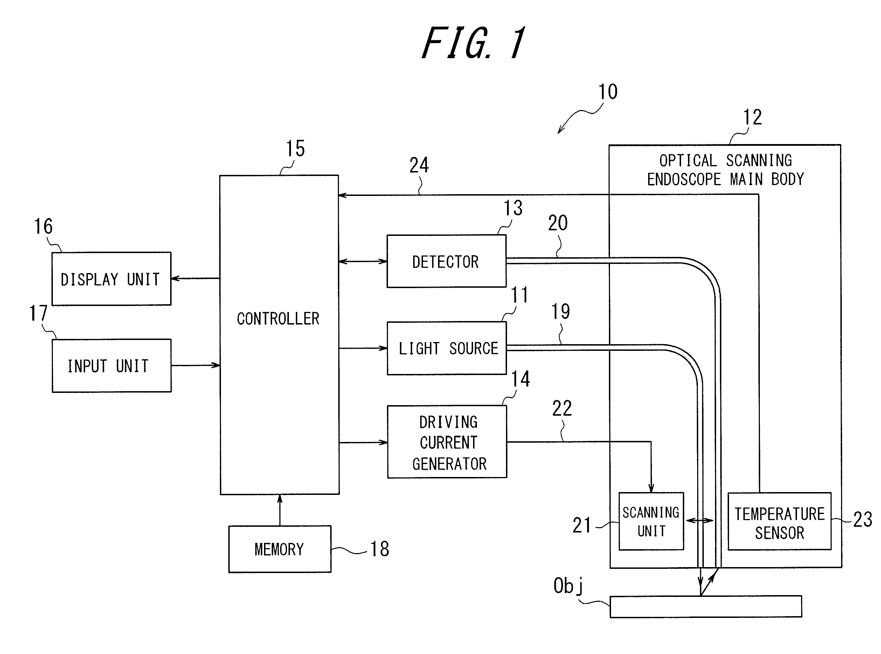

FIG. 1 is a functional block diagram schematically illustrating an internal configuration of an optical scanning observation apparatus according to Embodiment 1.

The optical scanning observation apparatus 10 is, for example, an optical scanning endoscope apparatus, and configured by including: a light source 11; an optical scanning endoscope main body 12; a detector 13; a driving current generator 14; a controller 15; a display unit 16; an input unit 17; and a memory 18.

The light source 11 and the optical scanning endoscope main body 12 are optically connected to each other via an illumination optical fiber 19 configured as a single mode fiber, and the detector 13 and the optical scanning endoscope main body 12 are optically connected to each other via a detection optical fiber bundle 20 having multimode fibers. Here, the light source 11, the detector 13, the driving current generator 14, the controller 15, and the memory 18 may all be accommodated in the same housing or may independently be accommodated in separate housings.

The light source 11 multiplexes light from three laser sources emitting CW (continuous wave) laser light in three primary colors of red, green, and blue, and emits the multiplexed light as white light. DPSS lasers (diode-pumped solid-state laser) and laser diodes, for example, may suitably be used as the laser source. It is obvious, however, that the configuration of the light source 11 is not limited thereto, and may use one laser light source or a plurality of other light sources.

The optical scanning endoscope main body 12 scans light emitted from the light source 11 via an illumination optical fiber 19, on an observation object *obj* by means of the scanning unit 21 (vibratory drive unit), condenses signal light obtained through the scanning, and transmits the condensed light to the detector 13 via the detection optical fiber bundle 20.

The detector 13 decompose the signal light having propagated through the detection optical fiber bundle 20, into spectral components, and converts the signal light into an electric signal by means of a photodetector using, for example, a photomultiplier tube or a photodiode.

The driving current generator 14 (vibratory drive unit) applies a vibration current, as a drive signal, to the scanning unit 21 via a wiring cable 22, based on control from the controller 15.

The controller 15 (changer) synchronously controls the light source 11, the detector 13, and the driving current generator 14, while processing electric signals output by the detector 13 so as to synthesize an image and displaying an image on the display unit 16. The controller 15 may also make, through the input unit 17, various settings in the optical scanning observation apparatus 10 as to the scanning speed and the brightness of an image to be displayed. Further, as will be described later, the controller 15 obtains temperature from a temperature sensor 23 disposed in the optical scanning endoscope main body 12, via a wiring cable 24, determines the frequency of the drive signal based on the temperature thus obtained, and causes the driving current generator 14 to change the frequency of the drive signal.

The memory 18 is, for example, a ROM, and stores predetermined information. For example, the memory 18 stores a correspondence table showing the relation between the frequency and the temperature, which is used for changing the frequency of the drive signal.

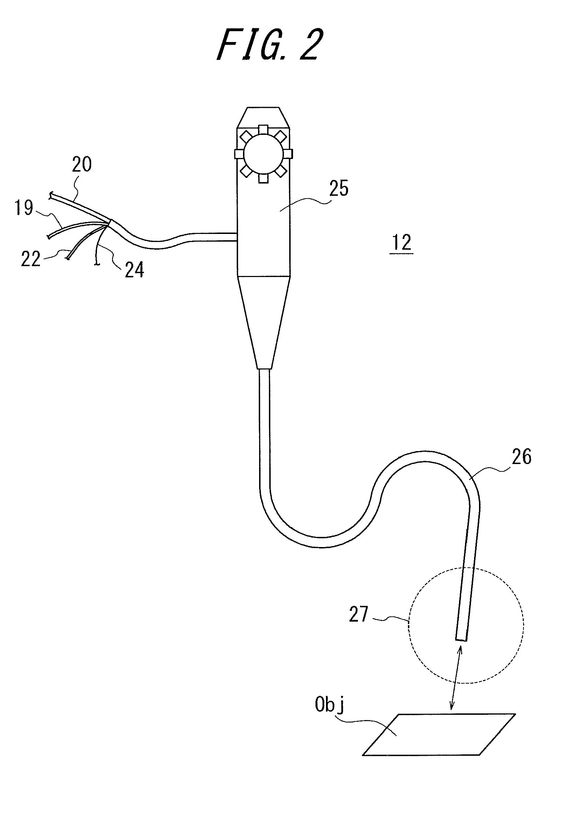

FIG. 2 is an overview schematically illustrating the optical scanning endoscope main body 12. The optical scanning endoscope main body 12 includes an operation portion 25 and an insertion portion 26, in which the operation portion 25 is connected at one end to the base end of the insertion portion 26 so as to be integrally formed therewith.

The operation portion 25 has the illumination optical fiber 19 from the light source 11, the detection optical fiber bundle 20 from the detector 13, the wiring cable 22 from the driving current generator 14, and the wiring cable 24 from the controller 15 each connected thereto. The illumination optical fiber 19, the detection optical fiber bundle 20; the wiring cable 22, and the wiring cable 24 are guided, through the inside of the insertion portion 26, to a tip part 27 (portion enclosed by the dashed line of FIG. 2) of the insertion portion 26.

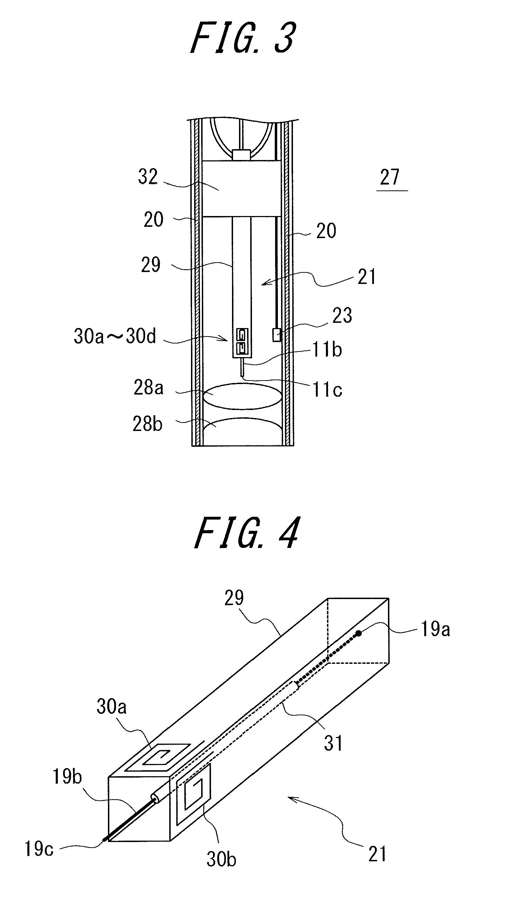

FIG. 3 is an enlarged sectional view of the tip part 27 of the optical scanning endoscope main body 12 of FIG. 12. The tip part 27 includes a scanning unit 21, a temperature sensor 23, projection lenses 28a, 28b, and a detection lens (not shown), while having the illumination optical fiber 19 and the detection optical fiber bundle 20 passing though the insertion portion 26 extending therethrough.

The scanning unit 21 is configured by including: an angular tube 29; deflection magnetic field generating coils 30a to 30d; and a permanent magnet 31 (see FIG. 4).

The angular tube 29 is in a rectangular hollow pillar shape, with one end closed and the other end open. The angular tube 29 is fixed within the tip part 27 by means of a mounting ring 32 in such a manner that the longitudinal direction of the tube coincides with the central axis of the tip part 27 while having the open end thereof facing toward the leading end side of the tip part 27 (see FIG. 3). Embodiment 1 employs the angular tube 29 that assumes a rectangular pillar shape, which however may assume a cylindrical shape or any other hollow shape.

The deflection magnetic field generating coils 30a to 30d are respectively disposed on each side surface of the angular tube 29 at the same position in the longitudinal direction (see FIG. 4). The deflection magnetic field generating coils 30a, 30c are disposed on mutually-opposing side surfaces, while the deflection magnetic field generating coil 30b, 30d are disposed on other mutually opposing side surfaces. A line passing through the centers of the deflection magnetic field generating coils 30a, 30c and a line passing through the centers of the deflection magnetic field generating coils 30b, 30d cross each other at right angles in the vicinity of the center axis of the angular tube 29. The deflection magnetic field generating coils 30a to 30d are connected to the driving current generator 14 via the wiring cable 22. The driving current generator 14 applies currents of independently different frequencies, as drive signals, to the deflection magnetic field generating coils 30a, 30c and to the deflection magnetic field generating coils 30b, 30d, respectively. Application of a drive signal to the deflection magnetic field generating coils 30a, 30c generates a first axis deflection magnetic field between the deflection magnetic field generating coils 30a and 30c. Application of a drive signal to the deflection magnetic field generating coils 30b, 30d generates a second axis deflection magnetic field between the deflection magnetic field generating coils 30a and 30c. The first axis deflection magnetic field and the second axis deflection magnetic field cause an oscillating part 19b having the permanent magnet 31, which is described later, to vibrate in two directions corresponding to the first axis deflection magnetic field and the second axis deflection magnetic field, according to the strength of each magnetic field.

The permanent magnet 31 is cylindrical in shape, and coupled with the illumination optical fiber 19 with the illumination optical fiber 19 being inserted through a through hole within the permanent magnet 31. The permanent magnet 31 is magnetized in the axial direction of the illumination optical fiber 19.

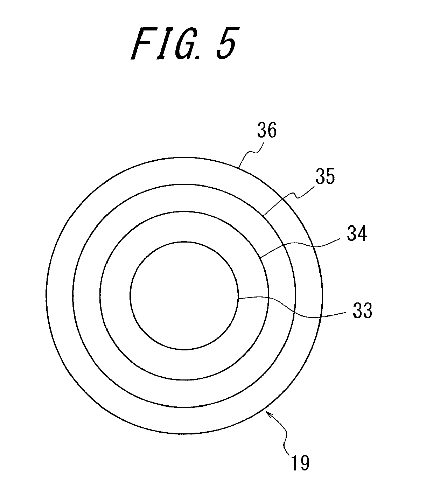

The illumination optical fiber 19 is composed of, as illustrated in FIG. 5, a core 33, a clad 34, and a coating 35. The core 33 is columnar in shape, and the clad 34 covers around the core 33. The coating 35 further covers around the clad 34. In part of the entire length of the illumination optical fiber 19, an optical fiber protection jacket 36 further coves around the coating 35. The core 33 includes at least one of: pure silica; fluorine-doped silica; and hydroxyl-doped silica, and is high in heat resistance and radiation resistance. The clad 34 includes at least one of: pure silica; fluorine-doped silica; and hydroxyl-doped silica, each having a refractive index lower than that of the core 33, and is high in heat resistance and radiation resistance. The coating 35 and the optical fiber protection jacket 36 each include at least one of: a polyimide resin; a polyethyletherketone resin; and an acrylate resin, and are high in heat resistance and radiation resistance.

The illumination optical fiber 19 is inserted through a hole formed in the closed end of the angular tube 29, and supported at the closed end of the angular tube 29 in such a manner that one pole of the permanent magnet 31 is interposed among the deflection magnetic field generating coils 30a to 30d (see FIG. 4). Therefore, the illumination optical fiber 19 is capable of oscillating from a fixed end 19a supported by the angular tube 29 to a tip 19c. A part of the illumination optical fiber 19 defined from the fixed end 19a including the permanent magnet 31 to the tip 19c is referred to as oscillating part 19b.

The detection optical fiber bundle 20 is formed of a plurality of detection optical fibers tied up in a bundle. Each of the detection optical fibers is composed of the core 33, the clad 34, the coating 35, and the optical fiber protection jacket 36, which are formed of members similar to those of the illumination optical fiber 19. Therefore, the detection optical fiber bundle 20 is, similarly to the illumination optical fiber 19, high in heat resistance and radiation resistance. The detection optical fiber bundle 20 is disposed so as to pass through the circumference of the tip part 27 of the insertion portion 26, and extends to the leading end of the tip part 27. The detection optical fiber bundle 20, which is configured to pass through the circumference of the tip part 27 in Embodiment 1, is not limited to such configuration, and may also be disposed as being in a bundle.

The temperature sensor 23 is fixed in the vicinity of the oscillating part 19b, within the tip part 27 of the insertion portion 26. The temperature sensor 23 is, for example, a thermocouple or a thermistor, and detects temperature near the oscillating part 19b.

The projection lenses 28a, 28b and the detection lens are arranged at the most leading end of the tip part 27 of the insertion portion 26. The projection lenses 28a, 28b are configured such that laser light emitted from the tip 19c of the illumination optical fiber 19 is substantially condensed onto an observation object *obj.* The detection lens is also arranged in such a manner as to take in, as detection light, the laser light that has been condensed onto the observation object *obj* and then reflected, scattered, and refracted by the observation object *obj* (light that has been interacted with the observation object *obj*) or fluorescence, so as to have the detection light condensed onto and coupled to the detection optical fiber bundle 20 disposed in the subsequent stage of the detection lens. The number of the projection lenses is not limited two, and the projection lens(es) may be composed of one lens or a plurality of lenses.

With the aforementioned configuration, when carrying out observation by using the optical scanning observation apparatus 10, the controller 15 controls the driving current generator 14 to be driven so as to apply, via the wiring cable 22, a vibration current serving as a drive signal to the deflection magnetic field generating coils 30a to 30d constituting the scanning unit 21, to thereby vibrate the oscillating part 19b.

The controller 15 causes the light source 11 to emit laser light, along with the application of a current by the driving current generator 14, and emits the laser light by means of the illumination optical fiber 19 from the tip 19c thereof toward the observation object *obj.* The vibration of the oscillating part 19b causes deflection in the tip 19c, to thereby sequentially scan laser light on the observation object *obj.*

The irradiation of laser light onto the observation object *obj* provides reflected light, scattered light, and light generated from the observation object *obj,* which are condensed by the detection lens as detection light and coupled to the detection optical fiber bundle 20. The detection light is guided through the detection optical fiber bundle 20 to the detector 13, and separated for each wavelength component and detected in the detector 13.

The controller 15 calculates, based on the phase of a drive signal applied by the driving current generator 14, information on the scanning position on the scanning path, while obtaining, based on an electric signal output from the detector 13, pixel data on the observation object *obj* at the scanning position. The controller 15 sequentially stores, in a frame memory, information on the scanning position and the pixel data, subjects the information to necessary processing such as interpolation processing after completing the scanning or during the scanning, so as to generate an image of the observation object *obj,* and displays the image on the display unit 16.

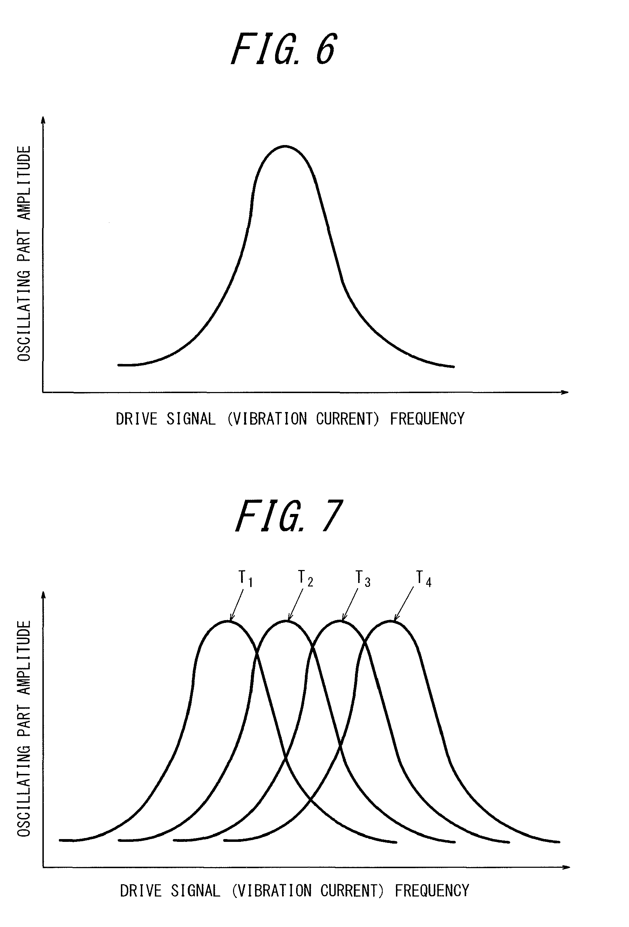

Next, description is given of how the controller 15 changes the frequency of a drive signal, based on the temperature in the vicinity of the oscillating part 19b. The oscillating part 19b vibrates at a frequency substantially the same as the frequency of a drive signal. The amplitude of the vibration of the oscillating part 19b varies depending on the frequency of the drive signal (see FIG. 6), and becomes maximum when the frequency of the drive signal is equal to or near the resonance frequency of the oscillating part 19b. In order to maximize the angle of view of the image to be picked up, the frequency of the drive signal can be matched to the frequency that maximizes the amplitude of the oscillating part 19b.

The resonance frequency is determined based on the dimensions, weight distribution, shape, elasticity, and the like of the oscillating part 19b, and thus, the resonance frequency may be caused to vary due to, for example, the dimensional variation resulting from temperature variation. Accordingly, when the resonance frequency of the oscillating part 19b varies due to changes in the external environment, the amplitude of the oscillating part 19b corresponding to the frequency of the drive signal may also vary. Therefore, a drive signal having a frequency that maximizes the amplitude at room temperature, for example, does not necessarily maximize the amplitude even in high temperatures, and the angle of view narrows along with the reduction in amplitude.

As shown in FIG. 7, for each of the temperatures (for example, T1, T2, T3, T4) in the vicinity of the oscillating part 19b, the frequency of the drive signal and the amplitude of the oscillating part 19b have a correspondence therebetween. The memory 18 stores, for each predetermined temperature range, the relation of the frequency of the drive signal that maximize the amplitude of the oscillating part 19b.

The controller 15 acquires a temperature in the vicinity of the oscillating part 19b detected by the temperature sensor 23, and reads out a frequency corresponding to the temperature, from the memory 18. The controller 15 causes the driving current generator 14 to generate a drive signal having the frequency thus read out.

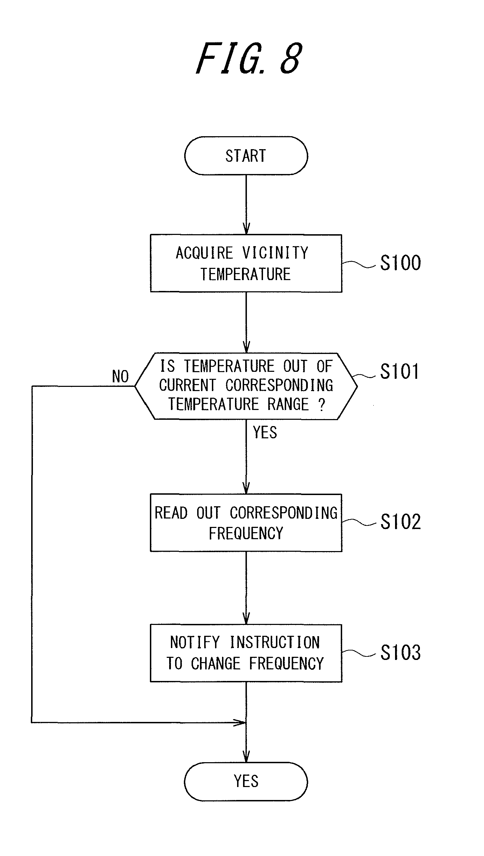

Next, in Embodiment 1, description is given of a process for determining the frequency of the drive signal which is carried out by the controller 15, with reference to the flowchart of FIG. 8. The processing of determining the frequency is carried out at predetermined intervals during the course of observing the observation object *obj* by the optical scanning observation apparatus 10. Alternatively, the process for determining the frequency may be started based on an input to the input unit 17 made by the user.

In Step S100, the controller 15 acquires, from the temperature sensor 23, a temperature in the vicinity of the oscillating part 19b. When the temperature in the vicinity is acquired, the process proceeds to Step S101.

In Step S101, the controller 15 determines whether the temperature in the vicinity of the oscillating part 19b acquired in Step S100 falls within a temperature range corresponding to the frequency of the current drive signal. When the temperature in the vicinity falls within the corresponding temperature range, the process proceeds to Step S102. When the temperature in the vicinity falls out of the corresponding temperature range, the process of determining the frequency is terminated.

In Step S102, the controller 15 reads out, from the memory 18, a frequency corresponding to a temperature range including the temperature in the vicinity of the oscillating part 19b acquired in Step S100. After reading out the frequency, the process proceeds to Step S103.

In Step S103, the controller 15 notifies the driving current generator 14 of an instruction to change the frequency of the drive signal to the frequency read out in Step S102. After the instruction to change the frequency is notified, the process for determining the frequency is terminated.

According to the optical scanning observation apparatus of Embodiment 1 configured as described above, which is capable of changing the frequency of the drive signal, narrowing of the angle of view can be suppressed, irrespective of changes in the external environment.

Further, according to the optical scanning observation apparatus of Embodiment 1, which changes the frequency of the drive signal based on the temperature in the vicinity of the oscillating part 19b detected by the temperature sensor 23, narrowing of the angle of view can be suppressed, irrespective of changes in the external environment, especially change in the temperature.

Still further, according to the optical scanning observation apparatus of Embodiment 1, in which the illumination optical fiber 19 and the detection optical fiber bundle 20 are both high in heat resistance and radiation resistance, deterioration and change in shape of the illumination optical fiber 19 and the detection optical fiber bundle 20 under high temperature environment and/or high dose of radiation can be suppressed.

Next, Embodiment 2 is described. In Embodiment 2, the method of changing the frequency of the drive signal is different from that of Embodiment 1. In the following, Embodiment 2 is described mainly about the differences from Embodiment 1. It should be noted that parts similar in configuration to those of Embodiment 1 are denoted by the same reference symbols.

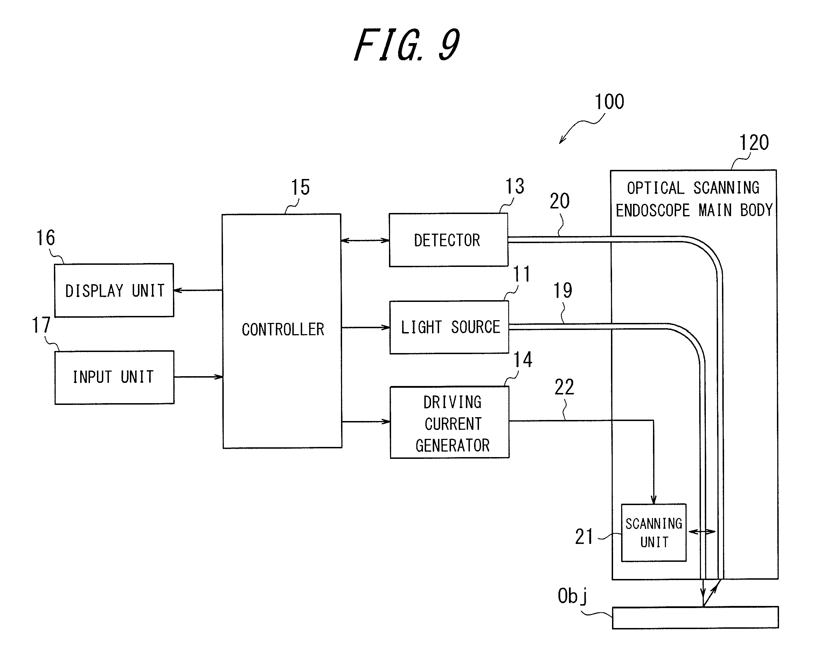

As illustrated in FIG. 9, in Embodiment 2, the optical scanning observation apparatus 100 is configured by including: a light source 11; an optical scanning endoscope main body 120; a detector 13; a driving current generator 14; a controller 15; a display unit 16; and the input unit 17 (see FIG. 9). The light source 11, the detector 13, the driving current generator 14, the display unit 16, and the input unit 17 are similar to those of Embodiment 1 in terms of configuration and function thereof.

Unlike Embodiment 1, the optical scanning endoscope main body 120 is not necessarily provided with a temperature sensor. The optical scanning endoscope main body 120 is similar in configuration and function to that of Embodiment 1, except in that the optical scanning endoscope main body 120 is not necessarily provided with a temperature sensor.

The controller 15 controls, as in Embodiment 1, the light source 11, the detector 13, and the driving current generator 14, to thereby synthesize an image and displays the image on the display unit 16. Further, the controller 15 can make various settings, as in Embodiment 1, in the optical scanning observation apparatus 100 as to the scanning speed and the brightness of an image to be displayed. Unlike in Embodiment 1, the controller 15 determines, when the input unit 17 detects an input of the frequency change made by the user, the frequency of the drive signal based on the input.

According to the optical scanning observation apparatus of Embodiment 2 configured as described above, which is also capable of changing the frequency of the drive signal, narrowing of the angle of view can be suppressed. Further, according to the optical scanning observation apparatus of Embodiment 2, in which the illumination optical fiber 19 and the detection optical fiber bundle 20 are again high in heat resistance and radiation resistance, deterioration and change in shape of the illumination optical fiber 19 and the detection optical fiber bundle 20 under high temperature environment and/or high dose of radiation can be suppressed.

While the disclosed apparatus has been described with reference to the accompanying drawings and Embodiments, various modifications and alternations can readily be made by those skilled in the art based on the disclosure. Therefore, such modifications and alterations fall within the scope of the present disclosure.

For example, in Embodiment 1 and Embodiment 2, the scanning unit 21 is configured to use the deflection magnetic field generating coils 30a to 30d and the permanent magnet 31 in order to vibrate the oscillating part, whereas any other drive means such as a piezoelectric element may also be used. In this regard, however, when the use under high temperature environment is assumed, it is preferred to use drive means that is excellent in temperature resistance, such as the combination of the deflection magnetic field generating coils 30a to 30d with the permanent magnet 31 as in Embodiment 1 and Embodiment 2. Further, the optical scanning observation apparatus disclosed herein is applicable not only to an optical scanning endoscope apparatus but also to any other apparatuses such as a microscope.

### REFERENCE SIGNS LIST

10, 100 optical scanning observation apparatus
11 light source
12, 120 optical scanning endoscope main body
13 detector
14 driving current generator
15 controller
16 display unit
17 input unit
18 memory
19 illumination optical fiber
19a fixed end
19b oscillating part
19c tip
20 detection optical fiber bundle
21 scanning unit
22 wiring cable
23 temperature sensor
24 wiring cable
25 operation portion
26 insertion portion
27 tip part
28a, 28b projection lens
29 angular tube
30a to 30d deflection magnetic field generating coil
31 permanent magnet
32 mounting ring
33 core
34 clad
35 coating
36 optical fiber protection jacket
*obj* observation object

## Claims

1. An optical scanning observation apparatus, comprising:
an optical fiber having an oscillating part supported at one end in an oscillatable manner;
a vibratory drive unit for vibrating the oscillating part, based on a drive signal; and
a controller for changing the frequency of the drive signal to be transmitted to the vibratory drive unit.

2. The optical scanning observation apparatus according to claim 1, further comprising a temperature sensor for detecting a temperature in the vicinity of the oscillating part,
wherein the controller changes the frequency of the drive signal, based on the temperature detected by the temperature sensor.

3. The optical scanning observation apparatus according to claim 2, further comprising a memory for storing the correspondence of the frequency to the temperature in the vicinity of the oscillating part,
wherein the controller reads out, from the memory, a frequency corresponding to the temperature detected by the temperature sensor, and makes the frequency of the drive signal consistent with the frequency thus read out.

4. The optical scanning observation apparatus according to any one of claims 1 to 3, wherein the optical fiber has: a core including at least one of pure silica, fluorine-doped silica, and hydroxyl-doped silica; a clad including at least one of pure silica, fluorine-doped silica, and hydroxyl-doped silica; a coating including at least one of a polyimide resin, a polyethyletherketone resin, and an acrylate resin; and a protection jacket including at least one of a polyimide resin, a polyethyletherketone resin, and an acrylate resin.

5. The optical scanning observation apparatus according to any one of claims 1 to 4, further comprising a detection optical fiber having: a core including at least one of pure silica, fluorine-doped silica, and hydroxyl-doped silica; a clad including at least one of pure silica, fluorine-doped silica, and hydroxyl-doped silica; a coating including at least one of a polyimide resin, a polyethyletherketone resin, and an acrylate resin; and a protection jacket including at least one of a polyimide resin, a polyethyletherketone resin, and an acrylate resin,
the detection optical fiber propagating light resulting from an interaction between light emitted from the oscillating part and the observation object.
